# EUROPÄISCHE PATENTSCHRIFT

(11) **EP 3 669 720 B1**
(45) Veröffentlichungstag und Bekanntmachung des Hinweises auf die Patenterteilung: **06.05.2026**
(21) Anmeldenummer: 19215314.6
(22) Anmeldetag: 11.12.2019
(51) Int. Cl.: A47J 36/32, G09B 19/00

(54) **LEBENSMITTELZUBEREITUNGSSYSTEM**
FOOD PREPARATION SYSTEM
SYSTÈME DE PRÉPARATION DE DENRÉES ALIMENTAIRES

(30) Priorität: 18.12.2018 ES 201831235
(43) Veröffentlichungstag der Anmeldung: 24.06.2020
(73) Patentinhaber: BSH Hausgeräte GmbH, 81739 München (DE)
(72) Erfinder: Franco Gutierrez, Carlos, 50017 Zaragoza (ES); Marzo Alvarez, Teresa Del Carmen, 50006 Zaragoza (ES); Paesa Garcia, David, 50015 Zaragoza (ES); Parra Borderías, Maria, 50006 Zaragoza (ES); Rodriguez Larrosa, Agostina, 50011 Zaragoza (ES); Villanueva Valero, Beatriz, 50011 Zaragoza (ES)

(56) Entgegenhaltungen:
- EP-B1- 2 870 470
- CN-A- 108 294 569
- CN-U- 205 018 795
- US-A1- 2016 198 885
- US-A1- 2016 213 033

## Beschreibung

Die Erfindung betrifft ein Lebensmittelzubereitungssystem nach dem Anspruch 1.

Aus dem europäischen Patent EP 2 870 470 B1 ist bereits ein Lebensmittelzubereitungssystem bekannt, welches eine Sensoreinheit aufweist. Die Sensoreinheit detektiert in einem Betriebszustand eine Sensorkenngröße aus einem Lebensmittelaufnahmeraum eines Gargeschirrs. Das Lebensmittelzubereitungssystem weist einen Löffel auf, in welchem die Sensoreinheit integriert ist. Zu einer Detektion der Sensorkenngröße muss der Löffel und damit die in dem Löffel integrierte Sensoreinheit manuell von einem Bediener in den Lebensmittelaufnahmeraum des Gargeschirrs eingebracht werden.

Die Dokumente CN 205018795 U und CN 108294569 A offenbaren ein Gargeschirr zur Erfassung des Nährstoffgehalts eines Lebensmittels.

Die Aufgabe der Erfindung besteht insbesondere darin, ein gattungsgemäßes System mit verbesserten Eigenschaften hinsichtlich eines Bedienkomforts bereitzustellen. Die Aufgabe wird erfindungsgemäß durch die Merkmale des Anspruchs 1 gelöst, während vorteilhafte Ausgestaltungen und Weiterbildungen der Erfindung den Unteransprüchen entnommen werden können.

Es wird ein Lebensmittelzubereitungssystem vorgeschlagen mit zumindest einem Gargeschirr, welches zumindest einen Lebensmittelaufnahmeraum zu einer Aufnahme zumindest eines Lebensmittels aufweist, mit zumindest einer Sensoreinheit, welche zu einer Detektion zumindest einer Sensorkenngröße aus dem Lebensmittelaufnahmeraum vorgesehen ist und welche in wenigstens einem Betriebszustand insbesondere zumindest eine Sensorkenngröße des, insbesondere in dem Lebensmittelaufnahmeraum befindlichen, Lebensmittels detektiert und welche wenigstens teilweise, insbesondere wenigstens zu einem Großteil und vorteilhaft vollständig in dem Gargeschirr integriert ist, und mit zumindest einer Steuereinheit, welche zu einer Ermittlung zumindest einer Komponente des Lebensmittels mittels der Sensoreinheit vorgesehen ist.

Durch die erfindungsgemäße Ausgestaltung kann insbesondere ein hoher Bedienkomfort erreicht werden. Insbesondere kann ein optimales Zubereitungsergebnis des Lebensmittels ermöglicht werden, und zwar insbesondere unabhängig von einem Erfahrungsgrad eines Bedieners und/oder auch im Fall zumindest einer für den Bediener neuen und/oder ungewohnten Zubereitungsart. Eine hohe Zufriedenheit bei einem Bediener und/oder eine Markentreue können/kann insbesondere bewirkt werden. Mittels der wenigstens teilweisen Integration der Sensoreinheit in dem Gargeschirr kann vorteilhaft eine Ausgabe eines hohen Informationsgrads an einem Bediener ermöglicht werden. Insbesondere kann in Abhängigkeit der Komponente des Lebensmittels zumindest eine Empfehlung hinsichtlich zumindest einer Zutat und/oder hinsichtlich zumindest eines Gewürzes bereitgestellt werden, und zwar insbesondere sowohl während einer Durchführung zumindest eines Rezepts als auch am Ende des Rezepts. Hierdurch können einem Bediener insbesondere selbst bei ihm unbekannten Rezepten, bei welchen der Bediener insbesondere wenig Erfahrung hat, optimale Zubereitungsergebnisse ermöglicht werden. Mittels der wenigstens teilweisen Integration der Sensoreinheit in dem Gargeschirr kann vorteilhaft eine durchgängige und/oder kontinuierliche Detektion der Sensorkenngröße ermöglicht werden, und zwar insbesondere automatisch und/oder unter Vermeidung einer Handlung eines Bedieners, welche beispielsweise ein Einbringen eines Löffels in den Lebensmittelaufnahmeraum sein könnte.

Unter einem "Lebensmittelzubereitungssystem" soll insbesondere ein System verstanden werden, welches zumindest eine Funktionseinheit aufweist, deren Hauptfunktion eine Zubereitung zumindest eines Lebensmittels und/oder zumindest eines Produkts ist, und welches insbesondere zusätzlich zumindest eine weitere Funktionseinheit aufweisen könnte, deren Hauptfunktion von einer Zubereitung zumindest eines Lebensmittels und/oder zumindest eines Produkts abweicht. Beispielsweise könnte die Funktionseinheit, deren Hauptfunktion insbesondere eine Zubereitung zumindest eines Lebensmittels und/oder zumindest eines Produkts ist, ein Gargerät und vorteilhaft ein Kochfeld sein. Alternativ oder zusätzlich könnte die Funktionseinheit, deren Hauptfunktion insbesondere eine Zubereitung zumindest eines Lebensmittels und/oder zumindest eines Produkts ist, insbesondere Teil eines Gargeräts und vorteilhaft eines Kochfelds sein. In wenigstens einem Betriebszustand erhitzt und/oder erwärmt und/oder gart die Funktionseinheit insbesondere zumindest ein Lebensmittel und/oder zumindest ein Produkt, und zwar insbesondere zum Zweck einer Zubereitung des Lebensmittels und/oder des Produkts.

Insbesondere führt die Funktionseinheit in wenigstens einem Betriebszustand zumindest eine Gargerätehauptfunktion und vorteilhaft zumindest eine Kochfeldhauptfunktion aus.

Insbesondere ist die Funktionseinheit Teil eines Gargeräts und vorteilhaft eines Kochfelds und führt in wenigstens einem Betriebszustand insbesondere zumindest eine Gargerätehauptfunktion des Gargeräts und vorteilhaft zumindest eine Kochfeldhauptfunktion des Kochfelds aus. Die Funktionseinheit könnte beispielsweise in wenigstens einem Betriebszustand zumindest eine Heizfunktion und/oder zumindest eine Garfunktion und/oder zumindest eine Kochfunktion ausführen.

Die weitere Funktionseinheit, deren Hauptfunktion insbesondere von einer Zubereitung zumindest eines Lebensmittels und/oder zumindest eines Produkts abweicht, könnte beispielsweise wenigstens teilweise in zumindest einem Mobilgerät und/oder in zumindest einem Haushaltsgerät, insbesondere in zumindest einem Kältegerät und/oder in zumindest einem Skalierungsgerät und/oder in zumindest einem Bearbeitungsgerät und/oder in zumindest einem Behandlungsgerät, integriert sein. Das Skalierungsgerät könnte insbesondere eine Waage, insbesondere eine Küchenwaage, sein. Das Bearbeitungsgerät könnte insbesondere eine Knetmaschine und/oder eine Teigmaschine und/oder ein Mixer und/oder eine Rührmaschine sein.

Die weitere Funktionseinheit könnte beispielsweise in wenigstens einem Betriebszustand zumindest eine Kühlungsfunktion und/oder zumindest eine Gefrierfunktion und/oder zumindest eine Bearbeitungsfunktion und/oder zumindest eine Behandlungsfunktion und/oder zumindest eine Skalierungsfunktion und/oder zumindest eine Reinigungsfunktion und/oder zumindest eine Trocknungsfunktion ausführen.

Insbesondere weist das Lebensmittelzubereitungssystem zumindest ein Gargerät und vorteilhaft zumindest ein Kochfeld auf. Das Gargeschirr ist insbesondere zu einer Beheizung durch das Gargerät und/oder durch das Kochfeld vorgesehen. Insbesondere ist das Gargerät und/oder das Kochfeld dazu vorgesehen, das Gargeschirr, insbesondere unabhängig von einer Positionierung des Gargeschirrs an dem Gargerät und/oder an dem Kochfeld, zu beheizen.

Das Gargeschirr ist insbesondere zu einem Aufstellen auf dem Kochfeld vorgesehen, und zwar insbesondere an einer beliebigen Position einer Kochfläche des Kochfelds. Insbesondere weist das Gargeschirr zumindest einen Gargeschirrgrundkörper auf, welcher den Lebensmittelaufnahmeraum insbesondere wenigstens teilweise begrenzt.

Das Gargeschirr weist insbesondere zumindest einen Gargeschirrdeckel auf, welcher insbesondere dem Gargeschirrgrundkörper zugeordnet ist und welcher insbesondere in wenigstens einem Betriebszustand den Lebensmittelaufnahmeraum wenigstens teilweise begrenzt. In wenigstens einem Betriebszustand begrenzt das Gargeschirr, insbesondere der Gargeschirrgrundkörper und der dem Gargeschirrgrundkörper zugeordnete Gargeschirrdeckel, den Lebensmittelaufnahmeraum insbesondere wenigstens zu einem Großteil, vorteilhaft wenigstens im Wesentlichen und besonders vorteilhaft vollständig.

Unter der Wendung, dass ein Objekt, insbesondere das Gargeschirr, vorteilhaft der Gargeschirrgrundkörper und/oder der Gargeschirrdeckel, einen Lebensmittelaufnahmeraum "wenigstens teilweise" begrenzt, soll insbesondere verstanden werden, dass das Objekt den Lebensmittelaufnahmeraum alleine oder gemeinsam mit zumindest einem weiteren Objekt, insbesondere dem Gargeschirrdeckel und/oder dem Gargeschirrgrundkörper, begrenzt. Unter "wenigstens zu einem Großteil" soll insbesondere zu einem Anteil, insbesondere einem Massenanteil und/oder einem Volumenanteil und/oder einem Anteil einer Anzahl, von mindestens 70 %, insbesondere von mindestens 80 %, vorteilhaft von mindestens 90 % und vorzugsweise von mindestens 95 % verstanden werden.

Das, insbesondere in dem Lebensmittelaufnahmeraum befindliche, Lebensmittel, welches insbesondere in dem Lebensmittelaufnahmeraum angeordnet ist, könnte beispielsweise zumindest einen Festkörper aufweisen. Vorzugsweise weist das, insbesondere in dem Lebensmittelaufnahmeraum befindliche, Lebensmittel zumindest ein Fluid auf und ist insbesondere wenigstens teilweise und vorteilhaft wenigstens zu einem Großteil als ein Fluid, insbesondere als das Fluid, ausgebildet und befindet sich insbesondere wenigstens teilweise und vorteilhaft wenigstens zu einem Großteil in fluidem Zustand. Unter der Wendung, dass das Lebensmittel "wenigstens teilweise" als zumindest ein Fluid ausgebildet ist, soll insbesondere verstanden werden, dass das Lebensmittel zu einem Anteil von mindestens 50 %, insbesondere von mindestens 60 %, vorteilhaft von mindestens 70 %, besonders vorteilhaft von mindestens 80 %, vorzugsweise von mindestens 90 % und besonders bevorzugt von mindestens 95 % als zumindest ein Fluid ausgebildet ist.

Unter einer "Sensoreinheit" soll insbesondere eine Einheit verstanden werden, welche zumindest einen Detektor zu einer Detektion wenigstens einer Sensorkenngröße, insbesondere der Sensorkenngröße, aufweist und welche insbesondere dazu vorgesehen ist, einen die Sensorkenngröße kennzeichnenden Wert auszugeben, wobei es sich bei der Sensorkenngröße vorteilhaft um eine physikalische und/oder chemische Größe handelt. Beispielsweise könnte die Sensoreinheit die Sensorkenngröße in wenigstens einem Betriebszustand aktiv detektieren, wie insbesondere durch Erzeugen und Aussenden eines Messsignals, insbesondere eines elektrischen und/oder optischen Messsignals. Alternativ oder zusätzlich könnte die Sensoreinheit die Sensorkenngröße in wenigstens einem Betriebszustand passiv detektieren, wie insbesondere durch eine Erfassung von zumindest einer Eigenschaftsänderung zumindest eines Sensorbauteils und/oder des Detektors. Vorteilhaft ist die Sensoreinheit zu einer Detektion zumindest einer Sensorkenngröße des, insbesondere in dem Lebensmittelaufnahmeraum befindlichen, Lebensmittels vorgesehen und detektiert in wenigstens einem Betriebszustand insbesondere zumindest eine Sensorkenngröße des, insbesondere in dem Lebensmittelaufnahmeraum befindlichen, Lebensmittels.

Die Sensorkenngröße unterscheidet sich insbesondere von einem Druck, insbesondere innerhalb des Lebensmittelaufnahmeraums. Insbesondere ist die Sensorkenngröße eine das Lebensmittel charakterisierende Kenngröße.

Unter der Wendung, dass die Sensoreinheit "wenigstens teilweise" in dem Gargeschirr integriert ist, soll insbesondere verstanden werden, dass die Sensoreinheit zumindest eine Detektionsstelle und/oder zumindest eine Sensorfläche und/oder zumindest eine Kommunikationseinheit und/oder zumindest einen Detektor aufweist, welche/welcher in dem Gargeschirr integriert ist. Insbesondere könnte die Sensoreinheit, insbesondere zusätzlich zu der Detektionsstelle und/oder zu der Sensorfläche und/oder zu der Kommunikationseinheit und/oder zu dem Detektor, zumindest ein Sensorelement aufweisen, welches außerhalb des Gargeschirrs angeordnet sein könnte. Das Sensorelement könnte beispielsweise zumindest eine weitere Detektionsstelle und/oder zumindest eine weitere Sensorfläche und/oder zumindest einen weiteren Detektor und/oder zumindest eine weitere Kommunikationseinheit aufweisen.

Unter einer "Steuereinheit" soll insbesondere eine elektronische Einheit verstanden werden, welche vorzugsweise dazu vorgesehen ist, zumindest die Sensoreinheit zu steuern und/oder zu regeln. Die Steuereinheit könnte beispielsweise in einer Steuer- und/oder Regeleinheit zumindest eines Haushaltsgeräts, insbesondere eines Gargeräts und vorteilhaft eines Kochfelds, wenigstens teilweise integriert sein und insbesondere dazu vorgesehen sein, zumindest eine Funktionseinheit des Haushaltsgeräts, insbesondere eines Gargeräts und vorteilhaft eines Kochfelds, welche insbesondere zu einer Durchführung zumindest einer Haushaltsgerätehauptfunktion insbesondere des Haushaltsgeräts, insbesondere zumindest einer Gargerätehauptfunktion insbesondere des Gargeräts und vorteilhaft zumindest einer Kochfeldhauptfunktion insbesondere des Kochfelds vorgesehen sein könnte, zu steuern und/oder zu regeln. Insbesondere weist die Steuereinheit eine Recheneinheit und insbesondere zusätzlich zur Recheneinheit eine Speichereinheit mit einem darin gespeicherten Steuer- und/oder Regelprogramm auf, das dazu vorgesehen ist, von der Recheneinheit ausgeführt zu werden.

Insbesondere detektiert die Steuereinheit in wenigstens einem Betriebszustand mittels der Sensoreinheit die Sensorkenngröße. Die Steuereinheit und die Sensoreinheit sind insbesondere zu einer, insbesondere drahtlosen, Kommunikation vorgesehen. Insbesondere weist die Sensoreinheit zumindest eine Kommunikationseinheit zu einer Kommunikation mit der Steuereinheit auf. Insbesondere weist die Steuereinheit zumindest eine Kommunikationseinheit zu einer Kommunikation mit der Sensoreinheit auf. Insbesondere hierbei, könnte die Steuereinheit insbesondere wenigstens teilweise in dem Gargerät, insbesondere in dem Kochfeld, und/oder in zumindest einem Mobilgerät, insbesondere des Lebensmittelzubereitungssystems, integriert sein.

Unter der Wendung, dass die Steuereinheit "mittels der Sensoreinheit" die Sensorkenngröße detektiert, soll insbesondere verstanden werden, dass die Steuereinheit zu der Detektion der Sensorkenngröße die Sensoreinheit ansteuert und insbesondere zumindest einen Befehl einer Detektion der Sensorkenngröße an die Sensoreinheit übermittelt. Insbesondere ermittelt die Steuereinheit in Abhängigkeit von der, insbesondere durch die Sensoreinheit detektierten, Sensorkenngröße die Komponente des Lebensmittels. Insbesondere führt die Steuereinheit zu der Ermittlung der Komponente des Lebensmittels zumindest eine Rechenoperation aus.

Die Komponente des Lebensmittels unterscheidet sich insbesondere von einem Druck, insbesondere innerhalb des Lebensmittelaufnahmeraums. Insbesondere ist die Komponente des Lebensmittels eine das Lebensmittel charakterisierende Komponente und/oder insbesondere Teil einer, insbesondere chemischen, Zusammensetzung des Lebensmittels. Die Komponente des Lebensmittels könnte beispielsweise zumindest ein Atom und/oder zumindest eine Atomzusammensetzung und/oder zumindest ein Molekül und/oder zumindest eine molekulare Teilzusammensetzung und/oder zumindest eine Geschmackskomponente des Lebensmittels aufweisen.

Unter "vorgesehen" soll insbesondere speziell programmiert, ausgelegt und/oder ausgestattet verstanden werden. Darunter, dass ein Objekt zu einer bestimmten Funktion vorgesehen ist, soll insbesondere verstanden werden, dass das Objekt diese bestimmte Funktion in zumindest einem Anwendungs- und/oder Betriebszustand erfüllt und/oder ausführt.

Beispielsweise könnte die Sensoreinheit wenigstens teilweise in dem Lebensmittelaufnahmeraum angeordnet sein und insbesondere zumindest ein Sensorelement und/oder zumindest einen Detektor aufweisen, welches/welcher in dem Lebensmittelaufnahmeraum angeordnet sein könnte. Die Sensoreinheit weist zumindest eine Sensorfläche auf, welche den Lebensmittelaufnahmeraum wenigstens abschnittsweise, insbesondere abschnittsweise, begrenzt. Unter einer "Sensorfläche" soll insbesondere eine Oberfläche zumindest eines Elements der Sensoreinheit verstanden werden, mittels welchem die Sensoreinheit insbesondere zu einer Detektion der Sensorkenngröße vorgesehen ist und/oder durch welches hindurch die Sensoreinheit insbesondere zu einer Detektion der Sensorkenngröße vorgesehen ist. Beispielsweise könnte das die Sensorfläche bildende und/oder definierende Element wenigstens zu einem Großteil aus zumindest einem transparenten Material bestehen und insbesondere wenigstens im Wesentlichen und vorteilhaft transparent ausgebildet sein. Das die Sensorfläche bildende und/oder definierende Element könnte beispielsweise wenigstens zu einem Großteil aus Kunststoff und/oder vorteilhaft aus Glas bestehen. Alternativ oder zusätzlich könnte das die Sensorfläche bildende und/oder definierende Element insbesondere zumindest eine Elektrode, insbesondere zumindest eine Sensorelektrode, aufweisen und/oder als Elektrode, insbesondere als Sensorelektrode, ausgebildet sein. Unter einer "Sensorelektrode" soll insbesondere zumindest ein Detektor der Sensoreinheit verstanden werden, welcher insbesondere in elektrisch leitendem Kontakt mit dem in dem Lebensmittelaufnahmeraum befindlichen Lebensmittel angeordnet und insbesondere zu einer Detektion der Sensorkenngröße vorgesehen ist. Unter der Wendung, dass die Sensorfläche den Lebensmittelaufnahmeraum "wenigstens abschnittsweise" begrenzt, soll insbesondere verstanden werden, dass die Sensorfläche zumindest einen Abschnitt des Lebensmittelaufnahmeraums begrenzt und insbesondere zumindest einen Teilbereich und/oder zumindest einen Abschnitt zumindest einer den Lebensmittelaufnahmeraum begrenzenden Wandung definiert und/oder ausbildet.

Die Sensorfläche begrenzt den Lebensmittelaufnahmeraum gemeinsam mit zumindest einer Gargeschirrwandung des Gargeschirrs und vorteilhaft zusätzlich mit zumindest einer weiteren Sensorfläche der Sensoreinheit. Die Sensoreinheit könnte beispielsweise zumindest zwei, insbesondere zumindest drei, vorteilhaft zumindest vier, besonders vorteilhaft zumindest fünf, vorzugsweise zumindest acht und besonders bevorzugt eine Vielzahl an Sensorflächen aufweisen. Dadurch kann insbesondere eine kompakte Ausgestaltung bei gleichzeitig optimalem Detektionsergebnis erzielt werden, wodurch insbesondere ein hoher Bedienkomfort und/oder ein qualitativ hochwertiges Gargerät, insbesondere Kochfeld, ermöglicht werden kann.

Zudem wird vorgeschlagen, dass die Steuereinheit dazu vorgesehen ist, zumindest ein bestimmtes Atom des Lebensmittels mittels der Sensoreinheit zu detektieren. Beispielsweise könnte die Steuereinheit in wenigstens einem Betriebszustand zumindest ein, insbesondere einzelnes, Atom und/oder zumindest eine, insbesondere einzige, Atomgruppe des Lebensmittels mittels der Sensoreinheit detektieren. Alternativ oder zusätzlich könnte die Steuereinheit in wenigstens einem Betriebszustand zumindest eine Atomzusammensetzung und/oder zumindest ein Molekül und/oder zumindest eine molekulare Teilzusammensetzung und/oder zumindest eine Stoffklasse des Lebensmittels mittels der Sensoreinheit detektieren. Beispielsweise könnte die Steuereinheit in wenigstens einem Betriebszustand, insbesondere einen Anteil an, Salz und/oder Glukose und/oder Ethanol, insbesondere des Lebensmittels, mittels der Sensoreinheit detektieren. Dadurch kann insbesondere eine hohe Detektionsauflösung ermöglicht werden, wodurch insbesondere eine qualitativ hochwertige und/oder genaue Detektion der Sensorkenngröße erzielt werden kann.

Die Steuereinheit ist dazu vorgesehen, zumindest eine bestimmte Geschmackskomponente des Lebensmittels mittels der Sensoreinheit zu detektieren. Unter einer "Geschmackskomponente" des Lebensmittels soll insbesondere zumindest ein Bestandteil zumindest eines Geschmacks verstanden werden. Beispielsweise könnte die Geschmackskomponente als "süß" und/oder "sauer" und/oder "bitter" und/oder "salzig" und/oder "unami" bezeichnet und insbesondere als Sinnesausdruck, insbesondere bei einer Nahrungsaufnahme, ausgebildet sein. Die Geschmackskomponente könnte beispielsweise durch zumindest ein Atom des Lebensmittels und/oder durch zumindest ein Molekül des Lebensmittels und/oder durch zumindest eine Mischung aus zumindest einem Atom des Lebensmittels und zumindest einem Molekül des Lebensmittels definiert und/oder gebildet sein. Vorteilhaft ist die Geschmackskomponente durch zumindest eine Mischung aus Atomen und/oder Molekülen definiert und/oder gebildet. In wenigstens einem Betriebszustand ermittelt die Steuereinheit insbesondere die Geschmackskomponente des Lebensmittels und/oder zumindest einen Geschmack des Lebensmittels auf Basis von zumindest einem Atom des Lebensmittels und/oder von zumindest einem Molekül des Lebensmittels und/oder von zumindest einer Mischung aus zumindest einem Atom des Lebensmittels und zumindest einem Molekül des Lebensmittels. Dadurch kann insbesondere ein geringes Risiko einer fehlerhaften Würzung des Lebensmittels erzielt und/oder eine Zubereitung zumindest eines geschmacklich den Bedürfnissen eines Bedieners entsprechenden Lebensmittels ermöglicht werden.

Die Sensoreinheit könnte beispielsweise zumindest ein, vorteilhaft genau ein, Sensorelement aufweisen, welches insbesondere zu der Detektion der Sensorkenngröße vorgesehen und insbesondere von zumindest einem, insbesondere von genau einem, Sensortyp sein könnte. Vorzugsweise weist die Sensoreinheit zumindest ein erstes Sensorelement und zumindest ein, insbesondere von dem ersten Sensorelement verschiedenes, zweites Sensorelement auf, welche von verschiedenem Sensortyp sind. Unter einem "Sensorelement" soll insbesondere ein Element der Sensoreinheit verstanden werden, welches zumindest einen Detektor der Sensoreinheit aufweist und welches insbesondere mittels des Detektors zu der Detektion der Sensorkenngröße vorgesehen ist und/oder welches in wenigstens einem Betriebszustand insbesondere mittels des Detektors die Sensorkenngröße detektiert. Zumindest eines der Sensorelemente könnte beispielsweise zumindest einen elektrochemischen Sensor aufweisen, wie beispielsweise zumindest einen potenziometrischen Sensor und/oder zumindest einen auf Voltammetrie basierenden Sensor und/oder zumindest einen impedimetrischen Sensor. Zumindest eines der Sensorelemente könnte beispielsweise zumindest einen Ionen-sensitiven Sensor aufweisen, wie beispielsweise zumindest einen als ISFET und/oder als ionensensitiver Feldeffekttransistor bezeichneten Sensor und/oder zumindest einen als ISE und/oder als ionensensitive Elektrode bezeichneten Sensor. Insbesondere könnte zumindest eines der Sensorelemente, insbesondere alternativ oder zusätzlich, zumindest einen Biosensor und/oder zumindest einen als "lab-on-a-chip" bezeichneten Sensor aufweisen.

Beispielsweise könnte die Steuereinheit in wenigstens einem Betriebszustand mittels jedes Sensorelements, insbesondere genau, eine Geschmackskomponente des Lebensmittels detektieren. Vorzugsweise detektiert die Steuereinheit in wenigstens einem Betriebszustand mittels jedes Sensorelements zumindest zwei, insbesondere zumindest drei, vorteilhaft zumindest vier und besonders vorteilhaft zumindest fünf Geschmackskomponenten des Lebensmittels. Insbesondere spricht zumindest eines der, vorteilhaft wenigstens ein Großteil der und vorzugsweise jedes der Sensorelemente auf zumindest zwei, insbesondere auf zumindest drei, vorteilhaft auf zumindest vier und besonders vorteilhaft auf zumindest fünf Geschmackskomponenten des Lebensmittels an. Beispielsweise könnte die Steuereinheit in wenigstens einem Betriebszustand zumindest eine bestimmte Geschmackskomponente des Lebensmittels, insbesondere die bestimmte Geschmackskomponente des Lebensmittels, mittels der Sensorelemente auf zumindest zwei verschiedene Arten detektieren. Beispielsweise könnte die Steuereinheit in wenigstens einem Betriebszustand zumindest eine als "salzig" bezeichnete Geschmackskomponente des Lebensmittels mittels der Sensorelemente, von welchen insbesondere zumindest eines zumindest einen Temperatursensor und zumindest ein weiteres zumindest einen elektrischen Sensor aufweist, auf zumindest zwei verschiedene Arten detektieren. Dadurch kann insbesondere eine hohe Flexibilität hinsichtlich der Detektion der Sensorkenngröße und/oder eine möglichst präzise Detektion der Sensorkenngröße ermöglicht werden, da insbesondere verschiedene Aspekte und/oder ein hoher Detektionsbereich abgedeckt werden können/kann.

Zudem wird vorgeschlagen, dass die Steuereinheit in wenigstens einem Betriebszustand zumindest mittels eines Ergebnisses einer Detektion der Sensorelemente, insbesondere mittels eines Ergebnisses einer Detektion des ersten Sensorelements und mittels eines Ergebnisses einer Detektion des zweiten Sensorelements, die Komponente des Lebensmittels ermittelt. Insbesondere berücksichtigt die Steuereinheit bei der Ermittlung der Komponente des Lebensmittels zumindest zwei, insbesondere zumindest drei, vorteilhaft zumindest vier, besonders vorteilhaft zumindest fünf und vorzugsweise mehrere Variablen. Zumindest zwei der Variablen könnten beispielsweise unabhängig voneinander sein. Alternativ oder zusätzlich könnten zumindest zwei der Variablen beispielsweise voneinander abhängig sein, und sich insbesondere wenigstens teilweise verstärken und/oder annullieren. Dadurch kann die Komponente des Lebensmittels insbesondere besonders genau ermittelt werden, wodurch insbesondere eine optimale Unterstützung eines Garprozesses ermöglicht werden kann.

Insbesondere weist das Lebensmittelzubereitungssystem zumindest eine Ausgabeeinheit auf, welche insbesondere zu einer Ausgabe zumindest einer Information, insbesondere an zumindest einen Bediener, vorgesehen ist. Unter einer "Ausgabeeinheit" soll insbesondere eine Einheit verstanden werden, welche dazu vorgesehen ist, einem Bediener insbesondere optisch und/oder akustisch und/oder haptisch zumindest eine Kenngröße bereitzustellen, wie beispielsweise eine Information und/oder eine Zeitangabe und/oder eine Bedienaufforderung und/oder eine Handlungsaufforderung und/oder eine Auswahl. Die Ausgabeeinheit könnte insbesondere dazu vorgesehen sein, wenigstens ein akustisches Signal und/oder insbesondere wenigstens eine akustische Sequenz auszugeben, wie beispielsweise einen Klingelton und/oder ein Warnsignal und/oder eine Aufforderung in Form eines insbesondere vorgefertigten Satzes. Alternativ oder zusätzlich könnte die Ausgabeeinheit dazu vorgesehen sein, eine optische Ausgabe bereitzustellen, wie beispielsweise eine Anzeige wenigstens eines Bilds und/oder wenigstens eines Texts und/oder wenigstens einer Ziffer und/oder wenigstens einer Animation. Beispielsweise könnte die Ausgabeeinheit zumindest einen Lautsprecher aufweisen. Alternativ oder zusätzlich könnte die Ausgabeeinheit eine Anzeigeeinheit, insbesondere mit zumindest einem Leuchtmittel, vorteilhaft einer LED, und/oder einem insbesondere hinterleuchteten Display, insbesondere einem Matrixdisplay und/oder einem LCD-Display, einem OLED-Display und/oder elektronischen Papier, aufweisen. Vorteilhaft weist die Ausgabeeinheit zumindest eine Flüssigkristallanzeige auf.

Das Lebensmittelzubereitungssystem weist insbesondere zumindest eine Bedienerschnittstelle auf, welche insbesondere zu einer Kommunikation, insbesondere der Steuereinheit, mit zumindest einem Bediener vorgesehen ist. Insbesondere ist die Bedienerschnittstelle zumindest zu einer Bedieneingabe insbesondere durch einen Bediener vorgesehen. Beispielsweise könnte die Bedienerschnittstelle zumindest ein Touch-Bedienelement aufweisen. Alternativ oder zusätzlich könnte die Bedienerschnittstelle zumindest ein akustisches Eingabeelement, wie beispielsweise zumindest ein Mikrophon, aufweisen, welches insbesondere zu einer akustischen Eingabe vorgesehen sein könnte. Die Bedienerschnittstelle könnte alternativ oder zusätzlich beispielsweise zumindest ein mechanisches Eingabeelement aufweisen, wie beispielsweise zumindest einen Joystick und/oder zumindest eine Tastatur und/oder zumindest eine Maus. Insbesondere könnten die Bedienerschnittstelle und die Ausgabeeinheit wenigstens teilweise einstückig miteinander ausgestaltet sein. Unter der Wendung, dass ein erstes Objekt und ein zweites Objekt "wenigstens teilweise einstückig" miteinander ausgestaltet sind, soll insbesondere verstanden werden, dass das erste Objekt zumindest ein Element aufweist, welches ebenfalls Teil des zweiten Objekts ist, und/oder dass das zweite Objekt zumindest ein Element aufweist, welches ebenfalls Teil des ersten Objekts ist. Insbesondere weisen die Bedienerschnittstelle und die Ausgabeeinheit jeweils zumindest ein, insbesondere zumindest zwei, vorteilhaft zumindest drei gemeinsame Elemente auf, die Bestandteil, insbesondere funktionell wichtiger Bestandteil, von sowohl der Bedienerschnittstelle als auch von der Ausgabeeinheit sind.

Weiterhin wird vorgeschlagen, dass die Steuereinheit dazu vorgesehen ist, insbesondere über die Ausgabeeinheit, durch zumindest ein Rezept zu führen und bei der Führung durch das Rezept die Komponente des Lebensmittels zumindest zu berücksichtigen. Dadurch kann insbesondere ein hoher Bedienkomfort erzielt werden. Insbesondere kann ein optimales Zubereitungsergebnis ermöglicht werden, da einem Bediener, insbesondere zusätzlich zu gängigen Informationen hinsichtlich Zeit und/oder Temperatur, vorteilhaft zumindest eine Information hinsichtlich eines Zubereitungsergebnisses, insbesondere hinsichtlich eines Geschmacks des zubereiteten Lebensmittels, bereitgestellt werden kann. Insbesondere kann einem Bediener eine Zubereitung eines optimal an seinen Geschmack angepassten Lebensmittels ermöglicht werden.

Unter einem "Rezept" soll insbesondere eine zeitliche Folge von Heizeinstellungen und/oder sonstigen Betriebseinstellungen und/oder Handlungsaufforderungen und/oder von Rezeptschritten zu einer Zubereitung wenigstens eines Lebensmittels und/oder wenigstens eines Garguts und/oder wenigstens einer Speise verstanden werden.

Das Rezept ist insbesondere als ein Kochrezept und/oder als ein Garrezept und/oder als ein Lebensmittelzubereitungsrezept ausgebildet. Insbesondere weist das Rezept zumindest zwei, insbesondere zumindest drei, vorteilhaft zumindest vier, besonders vorteilhaft zumindest fünf, vorzugsweise zumindest sechs und besonders bevorzugt mehrere Rezeptschritte auf.

Die Heizeinstellungen könnten beispielsweise wenigstens eine Heizdauer und/oder wenigstens eine Heizleistung und/oder wenigstens eine Heiztemperatur und/oder wenigstens eine Heizart, wie beispielsweise Braten und/oder Kochen und/oder Dämpfen und/oder Simmern und/oder Schmelzen und/oder Garen unter Druck, insbesondere in einem Druckkochtopf, und/oder Frittieren und/oder Sautieren und/oder Pochieren und/oder Backen und/oder Grillen, aufweisen. Die Betriebseinstellungen könnten beispielsweise zumindest eine Zeitdauer, welche eine Gesamtdauer des Rezepts definiert, und/oder zumindest eine zeitliche Abfolge von Rezeptschritten und/oder zumindest eine Art einer Ausgabe über die Ausgabeeinheit, wie beispielsweise optisch und/oder akustisch, aufweisen. Die Speise könnte insbesondere charakterisiert sein durch zumindest eine Zutatenzusammensetzung, welche insbesondere eine Gesamtheit aller zu der Zubereitung der Speise vorgesehenen Zutaten definieren könnte und/oder welche insbesondere durch alle zu der Zubereitung der Speise vorgesehenen Zutaten gekennzeichnet sein könnte, und zwar insbesondere in Zutatenquantität und/oder in Zutatenqualität und/oder in Lebensmitteltyp und/oder in Zutatentyp. Ein Weglassen zumindest einer Zutat und/oder eine Änderung zumindest einer Zutat könnte insbesondere in einer von der Speise verschiedenen weiteren Speise resultieren. Insbesondere könnte die Zutatenzusammensetzung eine Gesamtheit aller Zutaten sein, welche insbesondere zu einer gemeinsamen Garung und/oder Bearbeitung und/oder Zubereitung vorgesehen sein könnten und welche insbesondere alle in wenigstens einem Betriebszustand in wenigstens einem und vorteilhaft dem gleichen Gargeschirr angeordnet sein könnten.

Unter einem "Rezeptschritt" soll insbesondere ein Teilabschnitt eines Rezepts verstanden werden, welcher eine insbesondere definierte Zeitdauer und einen insbesondere definierten Verfahrensschritt aufweist, wie beispielsweise einen Heizschritt und/oder einen Schritt mit einer Interaktion über die Ausgabeeinheit und/oder einen Schritt mit einer Interaktion über die Bedienerschnittstelle und/oder einen Schritt mit einer Bearbeitung zumindest eines Lebensmittels und/oder einen Schritt mit einer Vorbereitung zumindest eines Lebensmittels, wobei die Zeitdauer insbesondere fest vorgegeben und/oder variabel, wie insbesondere von einer zu einer Bedieneingabe mittels der Bedienerschnittstelle benötigten Zeitdauer abhängig, ausgestaltet ist. Insbesondere könnte der Schritt mit einer Interaktion über die Ausgabeeinheit und/oder der Schritt mit einer Interaktion über die Bedienerschnittstelle als Interaktionsschritt bezeichnet sein. Insbesondere könnte der Schritt mit einer Bearbeitung zumindest eines Lebensmittels als Bearbeitungsschritt bezeichnet sein. Insbesondere könnte der Schritt mit einer Vorbereitung zumindest eines Lebensmittels als Lebensmittelvorbereitungsschritt bezeichnet sein. Der Rezeptschritt könnte beispielsweise ein Heizschritt und/oder ein Garschritt und/oder ein Lebensmittelvorbereitungsschritt und/oder ein Lebensmittelzugabeschritt und/oder ein Lebensmittelentnahmeschritt sein. Insbesondere unterscheidet sich der Rezeptschritt insbesondere von einer Abfrage einer Personenanzahl, für welche das Rezept insbesondere ausgelegt sein soll, und insbesondere von einer Abfrage einer Anzahl an Portionen, welche insbesondere mit dem Rezept zubereitet werden sollen.

Unter der Wendung, dass die Steuereinheit dazu vorgesehen ist, über die Ausgabeeinheit durch zumindest einen Rezeptschritt des Rezepts zu "führen", soll insbesondere verstanden werden, dass die Steuereinheit in wenigstens einem Betriebszustand mittels wenigstens einer Ausgabe über die Ausgabeeinheit zu zumindest einer Handlung auffordert und/oder zumindest eine Information ausgibt, und/oder dass die Steuereinheit in wenigstens einem Betriebszustand zu einer Durchführung des Rezepts notwendige Rezeptschritte automatisch vornimmt und/oder einleitet. Unter "automatisch" soll insbesondere selbsttätig und/oder unter Vermeidung einer Interaktion mittels der Bedienerschnittstelle und/oder unter Vermeidung einer Handlung eines Bedieners verstanden werden.

Insbesondere ist die Steuereinheit dazu vorgesehen, durch zumindest zwei, insbesondere durch zumindest drei, vorteilhaft durch zumindest fünf, besonders vorteilhaft durch zumindest acht, vorzugsweise durch zumindest zwölf und besonders bevorzugt durch eine Vielzahl an, insbesondere voneinander verschiedenen, Rezepten zu führen. Beispielsweise könnten zumindest zwei, insbesondere zumindest drei, vorteilhaft zumindest fünf, besonders vorteilhaft zumindest acht, vorzugsweise zumindest zwölf und besonders bevorzugt eine Vielzahl an, insbesondere voneinander verschiedenen, Rezepten in der Speichereinheit der Steuereinheit und/oder in zumindest einer Datenbank, insbesondere eines externen Servers, hinterlegt sein, auf welche die Steuereinheit insbesondere in wenigstens einem Betriebszustand zugreifen könnte.

Insbesondere könnte die von der Steuereinheit vorgenommene Ermittlung der Komponente des Lebensmittels in zumindest einem Rezept, vorteilhaft in wenigstens einem Großteil der Rezepte und vorzugsweise in jedem der Rezepte integriert sein.

Unter der Wendung, dass die Steuereinheit dazu vorgesehen ist, bei der Führung durch das Rezept die Komponente des Lebensmittels zumindest zu "berücksichtigen", soll insbesondere verstanden werden, dass die Steuereinheit bei der Führung durch das Rezept in Abhängigkeit von der Komponente des Lebensmittels zumindest eine Information und/oder zumindest eine Würzungsempfehlung für das Lebensmittel und/oder zumindest eine Bedienaufforderung zu einer Geschmacksprobe des Lebensmittels und/oder zumindest eine Bedienaufforderung hinsichtlich zumindest eines Feedbacks, insbesondere an einen Bediener und vorteilhaft über die Ausgabeeinheit und/oder über die Bedienerschnittstelle, ausgibt und/oder dass die Steuereinheit bei der Führung durch das Rezept in Abhängigkeit von der Komponente des Lebensmittels zumindest einen Rezeptschritt verändert und/oder anpasst und/oder überspringt und/oder abbricht.

Beispielsweise könnte die Steuereinheit in wenigstens einem Betriebszustand in Abhängigkeit der Komponente des Lebensmittels zumindest eine Empfehlung hinsichtlich zumindest einer Zutat und/oder hinsichtlich zumindest eines Gewürzes bereitstellen und/oder ausgeben, und zwar insbesondere sowohl während einer Durchführung zumindest eines Rezepts als auch am Ende des Rezepts. Die Steuereinheit könnte in wenigstens einem Betriebszustand insbesondere in Abhängigkeit der Komponente des Lebensmittels zumindest eine Konsistenz des Lebensmittels ermitteln und insbesondere in Abhängigkeit einer zeitlichen Änderung der Konsistenz des Lebensmittels zumindest eine Information, insbesondere hinsichtlich zumindest einer Zutat und/oder hinsichtlich zumindest einer Anpassung zumindest einer Zusammensetzung, insbesondere Zutatenzusammensetzung, des Lebensmittels, ausgeben.

Beispielsweise könnte die Steuereinheit, insbesondere alternativ oder zusätzlich, in wenigstens einem Betriebszustand in Abhängigkeit der Komponente des Lebensmittels zumindest eine Konsistenz des Lebensmittels ermitteln und, insbesondere automatisch, insbesondere zumindest einen, insbesondere aktuellen, Rezeptschritt des Rezepts beenden und, insbesondere automatisch, insbesondere zu zumindest einem, insbesondere nächstfolgenden, weiteren Rezeptschritt des Rezepts übergehen.

Ferner wird vorgeschlagen, dass die Steuereinheit in wenigstens einem Betriebszustand in Abhängigkeit von der Ermittlung der Komponente des Lebensmittels zumindest eine Information, insbesondere an einen Bediener und vorteilhaft über die Ausgabeeinheit und/oder über die Bedienerschnittstelle, ausgibt. Beispielsweise könnte die Steuereinheit in wenigstens einem Betriebszustand in Abhängigkeit von der Ermittlung der Komponente des Lebensmittels zumindest eine Information hinsichtlich zumindest einer Geschmackskomponente des Lebensmittels und/oder hinsichtlich zumindest einer Zutat zu einer Zubereitung des Lebensmittels, insbesondere an einen Bediener und vorteilhaft über die Ausgabeeinheit und/oder über die Bedienerschnittstelle, ausgeben. Dadurch kann einem Bediener insbesondere ein hoher Informationsgrad, insbesondere hinsichtlich des Lebensmittels und/oder hinsichtlich zumindest eines Rezepts, durch welches die Steuereinheit insbesondere den Bediener führt, bereitgestellt werden, wodurch der Bediener insbesondere bei der Zubereitung des Lebensmittels unterstützt werden kann, und zwar, insbesondere zusätzlich zu gängigen Informationen hinsichtlich Zeit und/oder Temperatur, vorteilhaft hinsichtlich eines Zubereitungsergebnisses, insbesondere hinsichtlich eines Geschmacks des zubereiteten Lebensmittels.

Beispielsweise könnte die Steuereinheit in wenigstens einem Betriebszustand zumindest eine Empfehlung hinsichtlich zumindest einer Zutat zu einer Zubereitung des Lebensmittels, insbesondere an einen Bediener und vorteilhaft über die Ausgabeeinheit und/oder über die Bedienerschnittstelle, ausgeben. Vorzugsweise gibt die Steuereinheit in wenigstens einem Betriebszustand zumindest eine Würzungsempfehlung für das Lebensmittel, insbesondere an einen Bediener und vorteilhaft über die Ausgabeeinheit und/oder über die Bedienerschnittstelle, aus. Insbesondere weist die Würzungsempfehlung zumindest eine Empfehlung hinsichtlich einer Zugabe zumindest eines Gewürzes zu dem Lebensmittel auf. Dadurch kann insbesondere ein geschmacklich optimiertes Zubereitungsergebnis des Lebensmittels erreicht werden, wodurch insbesondere eine hohe Zufriedenheit bei einem Bediener ermöglicht werden kann.

Weiterhin wird vorgeschlagen, dass die Steuereinheit in wenigstens einem Betriebszustand zumindest eine Bedienaufforderung, insbesondere an einen Bediener und vorteilhaft über die Ausgabeeinheit und/oder über die Bedienerschnittstelle, zu einer Geschmacksprobe des Lebensmittels ausgibt. Dadurch kann einem Bediener insbesondere vor Beendigung der Zubereitung des Lebensmittels zumindest eine Möglichkeit gegeben werden, eventuelle Korrekturen und/oder Änderungen einzuleiten und/oder ein Zubereitungsergebnis zu beeinflussen. Insbesondere kann hierdurch eine hohe Wahrscheinlichkeit eines optimal auf die Bedürfnisse des Bedieners angepassten Zubereitungsergebnisses erzielt werden.

Ferner wird vorgeschlagen, dass die Steuereinheit in wenigstens einem Betriebszustand zumindest eine Bedienaufforderung, insbesondere an einen Bediener und vorteilhaft über die Ausgabeeinheit und/oder über die Bedienerschnittstelle, hinsichtlich zumindest eines Feedbacks, insbesondere eines Bedieners, ausgibt. Beispielsweise könnte die Steuereinheit in dem Betriebszustand in Abhängigkeit des Feedbacks, insbesondere des Bedieners, zumindest ein neues Rezept, insbesondere mit zumindest einer neuen Komponente des Lebensmittels, abspeichern, und zwar insbesondere in der Speichereinheit und/oder in der Datenbank, insbesondere des externen Servers. Die Steuereinheit könnte in dem Betriebszustand, insbesondere alternativ oder zusätzlich, beispielsweise zeitlich vor der Speicherung als neues Rezept zumindest eine Bedienaufforderung hinsichtlich der Speicherung als neues Rezept ausgeben und insbesondere in Abhängigkeit einer Bedieneingabe hinsichtlich der Speicherung als neues Rezept zumindest ein neues Rezept, insbesondere mit zumindest einer neuen Komponente des Lebensmittels, abspeichern. Alternativ oder zusätzlich könnte die Steuereinheit in dem Betriebszustand insbesondere automatisch zumindest ein neues Rezept, insbesondere mit zumindest einer neuen Komponente des Lebensmittels, abspeichern. Insbesondere könnte die Steuereinheit in Abhängigkeit des Feedbacks, insbesondere des Bedieners, zumindest eine zukünftige Zubereitung des Lebensmittels an die Bedürfnisse des Bedieners anpassen und/oder das Feedback, insbesondere des Bedieners, bei zumindest einer zukünftigen Zubereitung des Lebensmittels berücksichtigen. Dadurch kann insbesondere eine Verbesserung der Zubereitung des Lebensmittels ermöglicht werden.

Zudem wird vorgeschlagen, dass die Steuereinheit dazu vorgesehen ist, in wenigstens einem Betriebszustand die Sensorkenngröße in, insbesondere regelmäßigen, zeitlichen Abständen von maximal 600 s, insbesondere von maximal 500 s, vorteilhaft von maximal 400 s, besonders vorteilhaft von maximal 300 s, vorzugsweise von maximal 200 s und besonders bevorzugt von maximal 120 s mittels der Sensoreinheit zu detektieren. Insbesondere ist die Steuereinheit dazu vorgesehen, in wenigstens einem Betriebszustand die Sensorkenngröße in, insbesondere regelmäßigen, zeitlichen Abständen von mindestens 1 s, insbesondere von mindestens 3 s, vorteilhaft von mindestens 5 s, besonders vorteilhaft von mindestens 10 s, vorzugsweise von mindestens 30 s und besonders bevorzugt von mindestens 60 s mittels der Sensoreinheit zu detektieren. Die Steuereinheit detektiert in dem Betriebszustand die Sensorkenngröße in den, insbesondere regelmäßigen, zeitlichen Abständen mittels der Sensoreinheit. Dadurch kann insbesondere flexibel auf Veränderungen in einer Zusammensetzung des Lebensmittels und/oder auf spontane Ereignisse reagiert werden, wodurch insbesondere eine hohe Flexibilität und/oder ein optimales Zubereitungsergebnis ermöglicht werden kann.

Die Steuereinheit könnte beispielsweise wenigstens teilweise als eine Mobilgeräte-Steuereinheit ausgebildet und insbesondere wenigstens teilweise, vorteilhaft wenigstens zu einem Großteil und besonders vorteilhaft vollständig in zumindest einem Mobilgerät, insbesondere des Lebensmittelzubereitungssystems, integriert sein. Beispielsweise könnte die Steuereinheit wenigstens teilweise als eine Gargeschirr-Steuereinheit ausgebildet und insbesondere wenigstens teilweise, vorteilhaft wenigstens zu einem Großteil und besonders vorteilhaft vollständig in dem Gargeschirr integriert sein. Vorzugsweise das Lebensmittelzubereitungssystem zumindest ein Gargerät, insbesondere zumindest ein Induktionsgargerät, vorteilhaft zumindest ein Kochfeld und vorzugsweise zumindest ein Induktionskochfeld, auf, welches die Steuereinheit aufweist. Vorteilhaft ist die Steuereinheit wenigstens teilweise als eine Geräte-Steuereinheit ausgebildet und insbesondere wenigstens teilweise, vorteilhaft wenigstens zu einem Großteil und besonders vorteilhaft vollständig in zumindest einem und insbesondere in zumindest einem Gerät integriert. Insbesondere ist die Geräte-Steuereinheit wenigstens teilweise, insbesondere wenigstens zu einem Großteil und vorteilhaft vollständig in zumindest einer Steuereinheit eines und insbesondere des Geräts integriert. Unter der Wendung, dass ein Objekt "wenigstens teilweise" als ein Geräte-Objekt ausgebildet ist, soll insbesondere verstanden werden, dass zumindest ein Teilobjekt des Objekts als Geräte-Teilobjekt ausgebildet ist und insbesondere in dem Geräte-Objekt integriert ist. Beispielsweise könnte zumindest ein weiteres Teilobjekt des Objekts als ein von einem Geräte-Objekt verschiedenes Geräte-Objekt ausgebildet sein, wie beispielsweise als ein Mobilgeräte-Objekt. Unter der Wendung, dass ein Objekt "als ein Geräte-Objekt ausgebildet" ist, soll insbesondere verstanden werden, dass das Objekt insbesondere wenigstens zu einem Großteil und vorteilhaft vollständig in zumindest einem Gerät integriert ist. Unter einem "Gerät" soll insbesondere ein Gargerät und/oder ein Induktionsgargerät und/oder ein Kochfeld und/oder ein Induktionskochfeld verstanden werden. Das Gargerät könnte beispielsweise zumindest ein Ofen, wie beispielsweise ein Herd und/oder ein Backofen, sein. Alternativ oder zusätzlich könnte das Gargerät beispielsweise eine Mikrowelle und/oder ein Grillgerät und/oder ein Dampfgarer sein. Vorteilhaft ist das Gargerät ein Kochfeld und vorzugsweise ein Induktionskochfeld. Dadurch kann insbesondere eine geringe Bauteilevielfalt und/oder eine geringe Lagerhaltung ermöglicht werden.

Das Lebensmittelzubereitungssystem soll hierbei nicht auf die oben beschriebene Anwendung und Ausführungsform beschränkt sein. Insbesondere kann das Lebensmittelzubereitungssystem zu einer Erfüllung einer hierin beschriebenen Funktionsweise eine von einer hierin genannten Anzahl von einzelnen Elementen, Bauteilen und Einheiten abweichende Anzahl aufweisen.

Weitere Vorteile ergeben sich aus der folgenden Zeichnungsbeschreibung. In der Zeichnung sind Ausführungsbeispiele der Erfindung dargestellt. Die Zeichnung, die Beschreibung und die Ansprüche enthalten zahlreiche Merkmale in Kombination. Der Fachmann wird die Merkmale zweckmäßigerweise auch einzeln betrachten und zu sinnvollen weiteren Kombinationen zusammenfassen.

Es zeigen:
- Fig. 1: ein Lebensmittelzubereitungssystem mit einem Gargeschirr und mit einem Gargerät in einer schematischen Draufsicht,
- Fig. 2: einen Ausschnitt des Lebensmittelzubereitungssystems mit dem Gargeschirr und mit dem Gargerät in einer schematischen Schnittdarstellung und
- Fig. 3: ein Diagramm einer Führung durch ein Rezept in einer schematischen Darstellung.

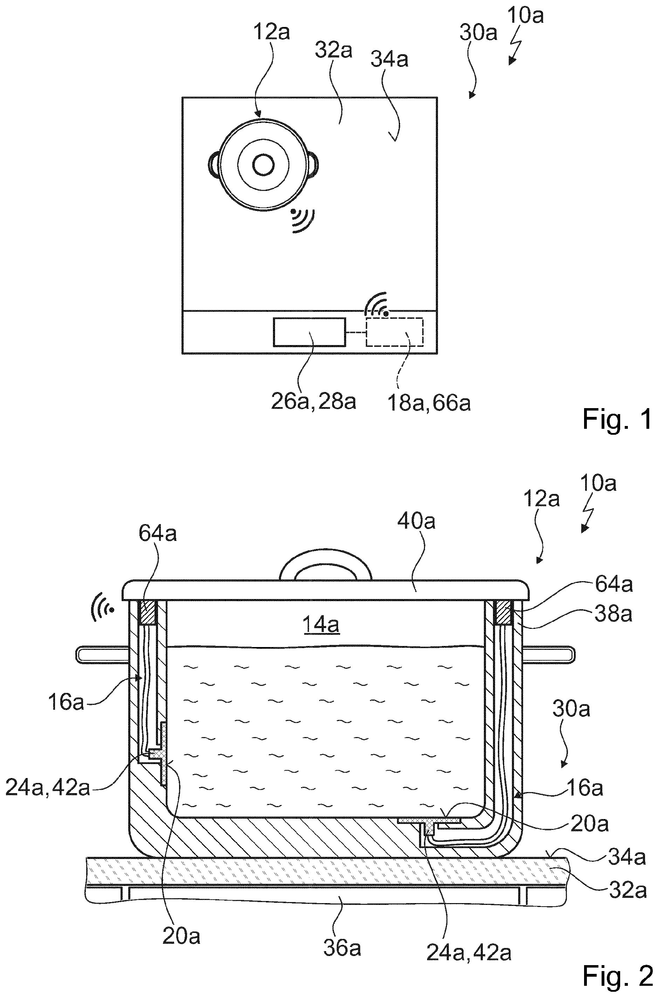

Figur 1 zeigt insbesondere ein Lebensmittelzubereitungssystem 10a, welches insbesondere ein Gargerät 30a aufweist. Das Gargerät 30a könnte beispielsweise zumindest ein Ofen, wie beispielsweise ein Herd und/oder ein Backofen, sein. Alternativ oder zusätzlich könnte das Gargerät 30a beispielsweise eine Mikrowelle und/oder ein Grillgerät und/oder ein Dampfgarer sein. Vorteilhaft ist das Gargerät 30a im vorliegenden Ausführungsbeispiel als ein Kochfeld ausgebildet. Das Gargerät 30a ist insbesondere als ein Induktionsgargerät ausgebildet. Insbesondere ist das Gargerät 30a im vorliegenden Ausführungsbeispiel als ein Induktionskochfeld ausgebildet. Vorteilhaft ist das Lebensmittelzubereitungssystem 10a im vorliegenden Ausführungsbeispiel als ein Kochsystem ausgebildet.

Das Gargerät 30a weist insbesondere zumindest eine und vorteilhaft genau eine Geräteplatte 32a auf. Im vorliegenden Ausführungsbeispiel ist die Geräteplatte 32a insbesondere als eine Aufstellplatte und vorteilhaft als eine Kochfeldplatte ausgebildet. In wenigstens einem montierten Zustand bildet die Geräteplatte 32a insbesondere einen Teil eines Geräteaußengehäuses, insbesondere eines Kochfeldaußengehäuses, aus, und zwar insbesondere eines Geräteaußengehäuses insbesondere des Gargeräts 30a und vorteilhaft eines Kochfeldaußengehäuses insbesondere des Kochfelds. Die Geräteplatte 32a ist im vorliegenden Ausführungsbeispiel insbesondere zu einem Aufstellen von Gargeschirr 12a vorgesehen.

Das Lebensmittelzubereitungssystem 10a weist insbesondere zumindest eine Heizeinheit 36a auf (vgl. Figur 2). Im vorliegenden Ausführungsbeispiel weist das Lebensmittelzubereitungssystem 10a insbesondere eine Vielzahl an Heizeinheiten 36a auf. Im Folgenden wird lediglich eine der Heizeinheiten 36a beschrieben. Die Heizeinheit 36a ist in einer Einbaulage insbesondere unterhalb der Geräteplatte 32a angeordnet. In wenigstens einem montierten Zustand ist die Heizeinheit 36a insbesondere in dem Gargerät 30a, vorteilhaft in dem Kochfeld, integriert. Die Heizeinheit 36a ist insbesondere dazu vorgesehen, auf der Geräteplatte 32a oberhalb der Heizeinheit 36a aufgestelltes Gargeschirr 12a zu erhitzen. Das Gargerät 30a, insbesondere das Kochfeld, weist insbesondere die Heizeinheit 36a auf.

Das Lebensmittelzubereitungssystem 10a weist insbesondere zumindest eine und vorteilhaft genau eine Bedienerschnittstelle 28a auf (vgl. Figur 1). Die Bedienerschnittstelle 28a ist insbesondere zu einer Eingabe und/oder Auswahl von Betriebsparametern vorgesehen, wie beispielsweise einer Heizleistung und/oder einer Heizleistungsdichte und/oder einer Heizzone. Die Bedienerschnittstelle 28a ist insbesondere zu einer Ausgabe, beispielsweise zu einer akustischen Ausgabe und vorteilhaft zu einer optischen Ausgabe, zumindest eines Betriebsparameters und/oder zumindest eines Werts eines Betriebsparameters, insbesondere an einen Bediener, vorgesehen. In wenigstens einem montierten Zustand ist die Bedienerschnittstelle 28a insbesondere in dem Gargerät 30a, vorteilhaft in dem Kochfeld, integriert. Das Gargerät 30a, insbesondere das Kochfeld, weist insbesondere die Bedienerschnittstelle 28a auf.

Das Lebensmittelzubereitungssystem 10a weist insbesondere zumindest eine und vorteilhaft genau eine Ausgabeeinheit 26a auf. Die Ausgabeeinheit 26a ist insbesondere wenigstens teilweise einstückig mit der Bedienerschnittstelle 28a ausgebildet. Insbesondere ist die Ausgabeeinheit 26a Teil der Bedienerschnittstelle 28a. Die Bedienerschnittstelle 28a weist insbesondere die Ausgabeeinheit 26a auf.

Die Ausgabeeinheit 26a ist insbesondere zu einer, insbesondere optischen, Ausgabe an einen Bediener vorgesehen. Im vorliegenden Ausführungsbeispiel weist die Ausgabeeinheit 26a insbesondere eine Flüssigkristallanzeige auf. Alternativ oder zusätzlich könnte die Ausgabeeinheit 26a beispielsweise zu einer akustischen und/oder haptischen Ausgabe an einen Bediener vorgesehen sein.

In wenigstens einem montierten Zustand ist die Ausgabeeinheit 26a insbesondere in dem Gargerät 30a, insbesondere in dem Kochfeld, integriert. Die Ausgabeeinheit 26a ist insbesondere Teil des Gargeräts 30a, insbesondere des Kochfelds. Alternativ könnte die Ausgabeeinheit 26a beispielsweise teilweise in dem Gargerät 30a und/oder insbesondere teilweise in zumindest einem Mobilgerät des Lebensmittelzubereitungssystems 10a und/oder vorteilhaft teilweise in dem Gargeschirr 12a integriert sein. Insbesondere weist das Gargerät 30a, insbesondere das Kochfeld, im vorliegenden Ausführungsbeispiel die Ausgabeeinheit 26a auf.

Das Lebensmittelzubereitungssystem 10a weist insbesondere zumindest eine und vorteilhaft genau eine Steuereinheit 18a auf. Die Steuereinheit 18a ist insbesondere dazu vorgesehen, in Abhängigkeit von mittels der Bedienerschnittstelle 28a eingegebener Betriebsparameter Aktionen auszuführen und/oder Einstellungen zu verändern. Die Steuereinheit 18a regelt in wenigstens einem Betriebszustand insbesondere eine Energiezufuhr zu der Heizeinheit 36a. In wenigstens einem montierten Zustand ist die Steuereinheit 18a insbesondere in dem Gargerät 30a, vorteilhaft in dem Kochfeld, integriert. Das Gargerät 30a, insbesondere das Kochfeld, weist insbesondere die Steuereinheit 18a auf.

Die Steuereinheit 18a ist, insbesondere mittels der Heizeinheit 36a, insbesondere zu einer Beheizung des Gargeschirrs 12a vorgesehen. Das Lebensmittelzubereitungssystem 10a weist insbesondere zumindest ein Gargeschirr 12a, vorteilhaft das Gargeschirr 12a, auf. Das Gargeschirr 12a weist insbesondere zumindest einen Lebensmittelaufnahmeraum 14a zu einer Aufnahme zumindest eines Lebensmittels auf (vgl. Figur 2). Das Gargeschirr 12a ist insbesondere zu einem Aufstellen auf der Geräteplatte 32a vorgesehen, und zwar insbesondere an einer beliebigen Position zumindest einer von der Geräteplatte 32a definierten Kochfläche 34a, insbesondere des Kochfelds.

Insbesondere weist das Gargeschirr 12a zumindest einen Gargeschirrgrundkörper 38a auf. Der Gargeschirrgrundkörper 38a begrenzt den Lebensmittelaufnahmeraum 14a insbesondere wenigstens teilweise. Das Gargeschirr 12a weist insbesondere zumindest einen Gargeschirrdeckel 40a auf. Der Gargeschirrdeckel 40a ist insbesondere dem Gargeschirrgrundkörper 38a zugeordnet. Insbesondere begrenzt der Gargeschirrdeckel 40a in dem Betriebszustand den Lebensmittelaufnahmeraum 14a wenigstens teilweise. In dem Betriebszustand begrenzt das Gargeschirr 12a, insbesondere der Gargeschirrgrundkörper 38a und der Gargeschirrdeckel 40a, den Lebensmittelaufnahmeraum 14a insbesondere wenigstens zu einem Großteil, vorteilhaft wenigstens im Wesentlichen und besonders vorteilhaft vollständig.

Das Lebensmittelzubereitungssystem 10a weist insbesondere zumindest eine und vorteilhaft genau eine Sensoreinheit 16a auf. Die Sensoreinheit 16a ist zu einer Detektion zumindest einer Sensorkenngröße aus dem Lebensmittelaufnahmeraum 14a vorgesehen, und zwar insbesondere in Abhängigkeit einer Ansteuerung durch die Steuereinheit 18a. Insbesondere detektiert die Steuereinheit 18a in dem Betriebszustand mittels der Sensoreinheit 16a die Sensorkenngröße aus dem Lebensmittelaufnahmeraum 14a.

In dem Betriebszustand detektiert die Steuereinheit 18a insbesondere die Sensorkenngröße in, insbesondere regelmäßigen, zeitlichen Abständen von maximal 120 s mittels der Sensoreinheit 16a. Insbesondere detektiert die Steuereinheit 18a in dem Betriebszustand die Sensorkenngröße in, insbesondere regelmäßigen, zeitlichen Abständen von mindestens 60 s mittels der Sensoreinheit 16a.

Besonders vorteilhaft ist die Sensoreinheit 16a wenigstens teilweise in dem Gargeschirr 12a integriert, und zwar insbesondere in dem Gargeschirrgrundkörper 38a des Gargeschirrs 12a. Die Sensoreinheit 16a begrenzt den Lebensmittelaufnahmeraum 14a insbesondere wenigstens abschnittsweise.

Insbesondere weist die Sensoreinheit 16a im vorliegenden Ausführungsbeispiel zumindest zwei, insbesondere zumindest drei, vorteilhaft zumindest vier, besonders vorteilhaft zumindest fünf und vorzugsweise mehrere Sensorflächen 20a auf. Von den Sensorflächen 20a sind in den Figuren insbesondere lediglich zwei dargestellt. Im Folgenden wird insbesondere lediglich eine der Sensorflächen 20a beschrieben.

Die Sensorfläche 20a begrenzt insbesondere den Lebensmittelaufnahmeraum 14a wenigstens abschnittsweise. Insbesondere weist die Sensoreinheit 16a, insbesondere pro Sensorfläche 20a, zumindest ein Element 42a auf, welches insbesondere die Sensorfläche 20a wenigstens teilweise, vorteilhaft wenigstens zu einem Großteil und vorzugsweise vollständig definiert und/oder ausbildet. Im Folgenden wird insbesondere lediglich eines der Elemente 42a beschrieben.

Insbesondere ist das Element 42a in dem Gargeschirrgrundkörper 38a und vorteilhaft in zumindest einer den Lebensmittelaufnahmeraum 14a begrenzenden Wandung des Gargeschirrgrundkörpers 38a integriert. Im vorliegenden Ausführungsbeispiel ist das Element 42a insbesondere als eine Elektrode ausgebildet.

Die Sensoreinheit 16a weist insbesondere zumindest ein erstes Sensorelement 22a und insbesondere zumindest ein zweites Sensorelement 24a auf. Insbesondere weist die Sensoreinheit 16a im vorliegenden Ausführungsbeispiel, insbesondere insgesamt, zumindest zwei, insbesondere zumindest drei, vorteilhaft zumindest vier, besonders vorteilhaft zumindest fünf und vorzugsweise mehrere Sensorelemente 22a, 24a auf. Von den Sensorelementen 22a, 24a sind in den Figuren insbesondere lediglich zwei dargestellt. Im Folgenden werden insbesondere lediglich zwei der Sensorelemente 22a, 24a beschrieben, und zwar insbesondere lediglich das erste Sensorelement 22a und das zweite Sensorelement 24a.

Insbesondere ist das erste Sensorelement 22a wenigstens teilweise und vorteilhaft vollständig einstückig mit dem Element 42a ausgebildet. Das erste Sensorelement 22a ist insbesondere als eine Elektrode ausgebildet.

Insbesondere ist das zweite Sensorelement 24a wenigstens teilweise und vorteilhaft vollständig einstückig mit dem Element 42a ausgebildet. Das zweite Sensorelement 24a ist insbesondere als eine Elektrode ausgebildet.

Das erste Sensorelement 22a und das zweite Sensorelement 24a sind vorteilhaft von verschiedenem Sensortyp. Beispielsweise könnte das erste Sensorelement 22a zumindest einen elektrochemischen Sensor aufweisen. Das zweite Sensorelement 24a könnte beispielsweise zumindest einen Ionen-sensitiven Sensor aufweisen. Insbesondere könnte, insbesondere alternativ oder zusätzlich zumindest eines der Sensorelemente 22a, 24a zumindest einen Biosensor und/oder zumindest einen als "lab-on-a-chip" bezeichneten Sensor aufweisen.

Die Steuereinheit 18a und die Sensoreinheit 16a sind insbesondere zu einer, insbesondere drahtlosen, Kommunikation vorgesehen. Insbesondere weist die Sensoreinheit 16a, insbesondere pro Sensorelement 22a, 24a, zumindest eine Kommunikationseinheit 64a zu einer Kommunikation mit der Steuereinheit 18a auf (vgl. Figur 2). Insbesondere weist die Steuereinheit 18a zumindest eine Kommunikationseinheit 66a zu einer Kommunikation mit der Sensoreinheit 16a auf (vgl. Figur 1).

Insbesondere ist die Steuereinheit 18a zu einer Ermittlung zumindest einer Komponente des Lebensmittels mittels der Sensoreinheit 16a vorgesehen, und zwar insbesondere auf Basis einer Detektion der Sensorkenngröße durch die Sensoreinheit 16a und/oder auf Basis eines Ergebnisses einer Detektion der Sensorelemente 22a, 24a. Die Steuereinheit 18a ermittelt in dem Betriebszustand insbesondere in Abhängigkeit einer Detektion der Sensorkenngröße durch die Sensoreinheit 16a die Komponente des Lebensmittels. Vorteilhaft ermittelt die Steuereinheit 18a in dem Betriebszustand zumindest mittels eines Ergebnisses einer Detektion der Sensorelemente 22a, 24a die Komponente des Lebensmittels.

Insbesondere detektiert die Steuereinheit 18a in dem Betriebszustand zumindest ein bestimmtes Atom des Lebensmittels mittels der Sensoreinheit 16a. In dem Betriebszustand detektiert die Steuereinheit 18a insbesondere zumindest eine bestimmte Geschmackskomponente des Lebensmittels mittels der Sensoreinheit 16a. Alterativ oder zusätzlich könnte die Steuereinheit 18a in dem Betriebszustand insbesondere in Abhängigkeit einer Detektion des Atoms des Lebensmittels die bestimmte Geschmackskomponente des Lebensmittels ermitteln.

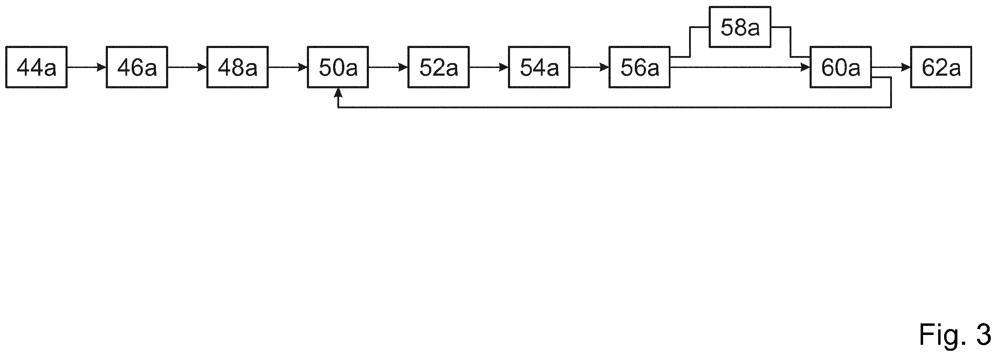

In dem Betriebszustand führt die Steuereinheit 18a insbesondere über die Ausgabeeinheit 26a, insbesondere einen Bediener, durch zumindest ein Rezept. Die Steuereinheit 18a berücksichtigt bei der Führung durch das Rezept die Komponente des Lebensmittels. Die Führung durch das Rezept wird im Folgenden anhand von Figur 3 beschreiben.

Insbesondere in zumindest einem Initialschritt 44a, ermittelt die Steuereinheit 18a in dem Betriebszustand insbesondere zumindest eine personenbezogene Kenngröße, insbesondere eines Bedieners. Im vorliegenden Ausführungsbeispiel ermittelt die Steuereinheit 18a in dem Betriebszustand die personenbezogene Kenngröße insbesondere mittels eines Benutzerprofils. Alternativ oder zusätzlich könnte die Steuereinheit 18a in dem Betriebszustand beispielsweise zumindest eine Bedienaufforderung, insbesondere über die Ausgabeeinheit 26a und/oder über die Bedienerschnittstelle 28a, ausgeben und die personenbezogene Kenngröße insbesondere mittels zumindest einer Bedieneingabe, insbesondere mittels der Bedienerschnittstelle 28a, ermitteln.

Beispielsweise könnte die personenbezogene Kenngröße zumindest eine Haltung eines Geschmacks und/oder zumindest eine Haltung hinsichtlich scharfer Gerichte und/oder zumindest eine Haltung hinsichtlich salziger Gerichte und/oder zumindest eine Haltung hinsichtlich einer Kalorienmenge aufweisen. Alternativ oder zusätzlich könnte die personenbezogene Kenngröße beispielsweise zumindest eine medizinische Kenngröße aufweisen, welche insbesondere zumindest eine Allergie eines Bedieners und/oder zumindest eine Lebensmittelunverträglichkeit eines Bedieners und/oder zumindest eine Krankheit eines Bedieners aufweisen könnte.

Insbesondere gibt die Steuereinheit 18a, insbesondere in zumindest einem Auswahlschritt 46a, in dem Betriebszustand zumindest eine Auswahl an Rezepten, insbesondere über die Ausgabeeinheit 26a und/oder über die Bedienerschnittstelle 28a, aus. In Abhängigkeit einer Bedieneingabe, insbesondere mittels der Bedienerschnittstelle 28a, wählt die Steuereinheit 18a in dem Betriebszustand, insbesondere in dem Auswahlschritt 46a, insbesondere zumindest ein Rezept aus.

Beispielsweise könnte die Steuereinheit 18a, insbesondere in zumindest einem Anpassungsschritt 48a, das, insbesondere ausgewählte, Rezept in Abhängigkeit von der personenbezogenen Kenngröße anpassen, wie beispielsweise zumindest einen Salzgehalt, insbesondere im Fall eines an zumindest einer Herzkrankheit und/oder zumindest einer Nierenkrankheit leidenden Bedieners, anpassen und/oder zumindest einen Anteil an Zucker, insbesondere im Fall eines an Diabetes leidenden Bedieners, anpassen und/oder zumindest einen Anteil an Pfeffer anpassen. Die Steuereinheit 18a könnte beispielsweise in dem Betriebszustand, insbesondere in dem Anpassungsschritt 48a, in Abhängigkeit von der personenbezogenen Kenngröße mittels der Sensoreinheit 16a zumindest einen, insbesondere gesundheitskritischen, Zutatenanteil des Lebensmittels überwachen und/oder kontrollieren und insbesondere zumindest ein gewünschtes Zubereitungsergebnis sichern. Der Zutatenanteil des Lebensmittels könnte beispielsweise Salz und/oder Zucker und/oder Pfeffer sein.

Alternativ oder zusätzlich, insbesondere zu der Anpassung des Rezepts, könnte die Steuereinheit 18a in dem Betriebszustand insbesondere zumindest eine Information, insbesondere an einen Bediener und vorteilhaft über die Ausgabeeinheit 26a und/oder über die Bedienerschnittstelle 28a, hinsichtlich zumindest einer alternativen Zubereitung und/oder hinsichtlich zumindest einer alternativen Zutat und/oder hinsichtlich zumindest einer alternativen Zutatenmenge ausgeben.

Insbesondere in zumindest einem Zugabeschritt 50a, fordert die Steuereinheit 18a in dem Betriebszustand insbesondere einen Bediener zu einer Zugabe zumindest einer Zutat in den Lebensmittelaufnahmeraum 14a auf. Insbesondere in Abhängigkeit von der Zugabe der Zutat in den Lebensmittelaufnahmeraum 14a geht die Steuereinheit 18a in zumindest einen Garschritt 52a über. In dem Garschritt 52a gart die Steuereinheit 18a, insbesondere mittels der Heizeinheit 36a, das in dem Lebensmittelaufnahmeraum 14a befindliche Lebensmittel.

Insbesondere in zumindest einem Prüfungsschritt 54a, prüft die Steuereinheit 18a in dem Betriebszustand, insbesondere in Abhängigkeit der Komponente des Lebensmittels, insbesondere ob eine bestimmte Geschmackskomponente des Lebensmittels und/oder eine bestimmte Konsistenz des Lebensmittels erreicht ist. In dem Betriebszustand gibt die Steuereinheit 18a, insbesondere in dem Prüfungsschritt 54a, in Abhängigkeit von der Ermittlung der Komponente des Lebensmittels zumindest eine Information, insbesondere an einen Bediener und vorteilhaft über die Ausgabeeinheit 26a und/oder über die Bedienerschnittstelle 28a, aus, und zwar insbesondere hinsichtlich zumindest einer bestimmten Geschmackskomponente des Lebensmittels und/oder hinsichtlich zumindest einer bestimmten Konsistenz des Lebensmittels und/oder hinsichtlich zumindest einer bestimmten Zutat und/oder Zutatenzusammensetzung des Lebensmittels.

Insbesondere in zumindest einem Testschritt 56a, gibt die Steuereinheit 18a in dem Betriebszustand, insbesondere über die Ausgabeeinheit 26a und/oder über die Bedienerschnittstelle 28a, zumindest eine Bedienaufforderung, insbesondere an einen Bediener, zu einer Geschmacksprobe des Lebensmittels aus. Die Steuereinheit 18a gibt in dem Betriebszustand, insbesondere in dem Testschritt 56a, zumindest eine Bedienaufforderung, insbesondere an einen Bediener und vorteilhaft über die Ausgabeeinheit 26a und/oder über die Bedienerschnittstelle 28a, hinsichtlich zumindest eines Feedbacks aus.

Insbesondere im Fall eines negativen Feedbacks, durch welches ein Bediener insbesondere seine Unzufriedenheit mit dem erreichten Garergebnis an die Steuereinheit 18a übermittelt, geht die Steuereinheit 18a in dem Betriebszustand insbesondere in zumindest einen Würzungsschritt 58a über. Insbesondere in dem Würzungsschritt 58a, gibt die Steuereinheit 18a in dem Betriebszustand insbesondere zumindest eine Würzungsempfehlung für das Lebensmittel, insbesondere an einen Bediener und vorteilhaft über die Ausgabeeinheit 26a und/oder über die Bedienerschnittstelle 28a, aus. Insbesondere geht die Steuereinheit 18a, insbesondere in Abhängigkeit einer Bedieneingabe hinsichtlich der Würzungsempfehlung, in zumindest einen, insbesondere weiteren, Prüfungsschritt 60a über.

Insbesondere im Fall eines positiven Feedbacks, durch welches ein Bediener insbesondere seine Zufriedenheit mit dem erreichten Garergebnis an die Steuereinheit 18a übermittelt, geht die Steuereinheit 18a in dem Betriebszustand insbesondere in den, insbesondere weiteren, Prüfungsschritt 60a über.

In dem Betriebszustand prüft die Steuereinheit 18a, insbesondere in dem, insbesondere weiteren, Prüfungsschritt 60a, das Rezept hinsichtlich einer Fertigstellung. Insbesondere im Fall einer Feststellung zumindest eines weiteren verbleibenden Rezeptschritts geht die Steuereinheit 18a in dem Betriebszustand insbesondere in den Zugabeschritt 50a über.

Insbesondere im Fall einer Feststellung einer Fertigstellung des Rezepts geht die Steuereinheit 18a in dem Betriebszustand insbesondere in zumindest einen Abschlussschritt 62a über. Insbesondere in dem Abschlussschritt 62a, gibt die Steuereinheit 18a in dem Betriebszustand, insbesondere über die Ausgabeeinheit 26a und/oder über die Bedienerschnittstelle 28a, zumindest eine Bedienaufforderung, insbesondere an einen Bediener, zu einer Geschmacksprobe des Lebensmittels aus. Die Steuereinheit 18a gibt in dem Betriebszustand, insbesondere in dem Abschlussschritt 62a, zumindest eine Bedienaufforderung, insbesondere an einen Bediener und vorteilhaft über die Ausgabeeinheit 26a und/oder über die Bedienerschnittstelle 28a, hinsichtlich zumindest eines Feedbacks aus.

In dem Betriebszustand speichert die Steuereinheit 18a insbesondere in Abhängigkeit des Feedbacks des Bedieners zumindest ein neues Rezept, insbesondere mit einer neuen Komponente des Lebensmittels, vorteilhaft in zumindest einer Speichereinheit der Steuereinheit 18a ab (nicht dargestellt). Insbesondere übermittelt ein Bediener mittels des Feedbacks des Bedieners zumindest eine Zustimmung zu einer Speicherung des neuen Rezepts.

### Bezugszeichen

- 10: Lebensmittelzubereitungssystem
- 12: Gargeschirr
- 14: Lebensmittelaufnahmeraum
- 16: Sensoreinheit
- 18: Steuereinheit
- 20: Sensorfläche
- 22: Erstes Sensorelement
- 24: Zweites Sensorelement
- 26: Ausgabeeinheit
- 28: Bedienerschnittstelle
- 30: Gargerät
- 32: Geräteplatte
- 34: Kochfläche
- 36: Heizeinheit
- 38: Gargeschirrgrundkörper
- 40: Gargeschirrdeckel
- 42: Element
- 44: Initialschritt
- 46: Auswahlschritt
- 48: Anpassungsschritt
- 50: Zugabeschritt
- 52: Garschritt
- 54: Prüfungsschritt
- 56: Testschritt
- 58: Würzungsschritt
- 60: Prüfungsschritt
- 62: Abschlussschritt
- 64: Kommunikationseinheit
- 66: Kommunikationseinheit

## Patentansprüche

1. Lebensmittelzubereitungssystem mit zumindest einem Gargeschirr (12a), welches zumindest einen Lebensmittelaufnahmeraum (14a) zu einer Aufnahme zumindest eines Lebensmittels aufweist, mit zumindest einer Sensoreinheit (16a), welche zu einer Detektion zumindest einer Sensorkenngröße aus dem Lebensmittelaufnahmeraum (14a) vorgesehen ist und welche wenigstens teilweise in dem Gargeschirr (12a) integriert ist, und mit zumindest einer Steuereinheit (18a), welche zu einer Ermittlung zumindest einer Komponente des Lebensmittels mittels der Sensoreinheit (16a) vorgesehen ist, wobei die Sensoreinheit (16a) zumindest eine Sensorfläche (20a) aufweist, welche den Lebensmittelaufnahmeraum (14a) gemeinsam mit zumindest einer Gargeschirrwandung des Gargeschirrs (12a) begrenzt, **dadurch gekennzeichnet, dass** die Steuereinheit (18a) dazu vorgesehen ist, zumindest eine bestimmte Geschmackskomponente des Lebensmittels mittels der Sensoreinheit (16a) zu detektieren.

2. Lebensmittelzubereitungssystem nach Anspruch 1, **dadurch gekennzeichnet, dass** die Steuereinheit (18a) dazu vorgesehen ist, zumindest ein bestimmtes Atom des Lebensmittels mittels der Sensoreinheit (16a) zu detektieren.

3. Lebensmittelzubereitungssystem nach Anspruch 1 oder 2, **dadurch gekennzeichnet, dass** die Sensoreinheit (16a) zumindest ein erstes Sensorelement (22a) und zumindest ein zweites Sensorelement (24a) aufweist, welche von verschiedenem Sensortyp sind.

4. Lebensmittelzubereitungssystem nach Anspruch 3, **dadurch gekennzeichnet, dass** die Steuereinheit (18a) in wenigstens einem Betriebszustand zumindest mittels eines Ergebnisses einer Detektion der Sensorelemente (22a, 24a) die Komponente des Lebensmittels ermittelt.

5. Lebensmittelzubereitungssystem nach einem der vorhergehenden Ansprüche, **dadurch gekennzeichnet, dass** die Steuereinheit (18a) dazu vorgesehen ist, durch zumindest ein Rezept zu führen und bei der Führung durch das Rezept die Komponente des Lebensmittels zumindest zu berücksichtigen.

6. Lebensmittelzubereitungssystem nach einem der vorhergehenden Ansprüche, **dadurch gekennzeichnet, dass** die Steuereinheit (18a) in wenigstens einem Betriebszustand in Abhängigkeit von der Ermittlung der Komponente des Lebensmittels zumindest eine Information ausgibt.

7. Lebensmittelzubereitungssystem nach einem der vorhergehenden Ansprüche, **dadurch gekennzeichnet, dass** die Steuereinheit (18a) in wenigstens einem Betriebszustand zumindest eine Würzungsempfehlung für das Lebensmittel ausgibt.

8. Lebensmittelzubereitungssystem nach einem der vorhergehenden Ansprüche, **dadurch gekennzeichnet, dass** die Steuereinheit (18a) in wenigstens einem Betriebszustand zumindest eine Bedienaufforderung zu einer Geschmacksprobe des Lebensmittels ausgibt.

9. Lebensmittelzubereitungssystem nach einem der vorhergehenden Ansprüche, **dadurch gekennzeichnet, dass** die Steuereinheit (18a) in wenigstens einem Betriebszustand zumindest eine Bedienaufforderung hinsichtlich zumindest eines Feedbacks ausgibt.

10. Lebensmittelzubereitungssystem nach einem der vorhergehenden Ansprüche, **dadurch gekennzeichnet, dass** die Steuereinheit (18a) dazu vorgesehen ist, in wenigstens einem Betriebszustand die Komponente des Lebensmittels in zeitlichen Abständen von maximal 600 s mittels der Sensoreinheit (16a) zu detektieren.

11. Lebensmittelzubereitungssystem nach einem der vorhergehenden Ansprüche, **gekennzeichnet durch** zumindest ein Gargerät (30a), welches die Steuereinheit (18a) aufweist.

## Claims

1. Food preparation system with at least one item of cookware (12a), which has at least one food receiving compartment (14a) for receiving at least one food, with at least one sensor unit (16a) which is provided to detect at least one sensor variable from the food receiving compartment (14a) and which is integrated at least partially in the item of cookware (12a), and with at least one control unit (18a), which is provided to determine at least one component of the food by means of the sensor unit (16a), wherein the sensor unit (16a) has at least one sensor surface (20a), which delimits the food receiving compartment (14a) together with at least one wall of the item of cookware (12a), **characterised in that** the control unit (18a) is provided to detect at least one specific taste component of the food by means of the sensor unit (16a).

2. Food preparation system according to claim 1, **characterised in that** the control unit (18a) is provided to detect at least one specific atom of the food by means of the sensor unit (16a).

3. Food preparation system according to claim 1 or 2, **characterised in that** the sensor unit (16a) has at least one first sensor element (22a) and at least one second sensor element (24a), which are of a different sensor type.

4. Food preparation system according to claim 3, **characterised in that** in at least one operating state, the control unit (18a) determines the component of the food at least by means of a result of a detection of the sensor elements (22a, 24a).

5. Food preparation system according to one of the preceding claims, **characterised in that** the control unit (18a) is provided to guide through at least one recipe and during the guidance through the recipe to at least take into account the component of the food.

6. Food preparation system according to one of the preceding claims, **characterised in that** in at least one operating state the control unit (18a) outputs at least one item of information as a function of the determination of the component of the food.

7. Food preparation system according to one of the preceding claims, **characterised in that** in at least one operating state the control unit (18a) outputs at least one seasoning recommendation for the food.

8. Food preparation system according to one of the preceding claims, **characterised in that** in at least one operating state the control unit (18a) outputs at least one control prompt with respect to a taste test of the food.

9. Food preparation system according to one of the preceding claims, **characterised in that** in at least one operating state the control unit (18a) outputs at least one control prompt in respect of at least one feedback.

10. Food preparation system according to one of the preceding claims, **characterised in that** in at least one operating state the control unit (18a) is provided to detect the component of the food at time intervals of at most 600 s by means of the sensor unit (16a).

11. Food preparation system according to one of the preceding claims, **characterised by** at least one cooking appliance (30a) which has the control unit (18a).

## Revendications

1. Système de préparation de denrées alimentaires comprenant :
au moins un ustensile de cuisson (12a), qui comprend au moins un espace de réception de denrées alimentaires (14a) destiné à recevoir au moins une denrée alimentaire,
au moins une unité de détection (16a), qui est configurée pour détecter au moins une grandeur caractéristique de détection à partir de l'espace de réception de denrées alimentaires (14a), et qui est intégrée au moins en partie dans l'ustensile de cuisson (12a), et
au moins une unité de commande (18a), qui est configurée pour déterminer au moins une composante de la denrée alimentaire au moyen de l'unité de détection (16a), dans lequel l'unité de détection (16a) comprend au moins une surface de détection (20a), qui délimite l'espace de réception de denrées alimentaires (14a) conjointement avec au moins une paroi d'ustensile de cuisson de l'ustensile de cuisson (12a), **caractérisé en ce que** l'unité de commande (18a) est configurée pour détecter au moins une composante gustative particulière de la denrée alimentaire au moyen de l'unité de détection (16a).

2. Système de préparation de denrées alimentaires selon la revendication 1, **caractérisé en ce que** l'unité de commande (18a) est conçue pour détecter au moins un atome particulier de la denrée alimentaire au moyen de l'unité de détection (16a).

3. Système de préparation de denrées alimentaires selon la revendication 1 ou 2, **caractérisé en ce que** l'unité de détection (16a) comprend au moins un premier élément de capteur (22a) et au moins un deuxième élément de capteur (24a), qui sont d'un type de capteur différent.

4. Système de préparation de denrées alimentaires selon la revendication 3, **caractérisé en ce que** l'unité de commande (18a) détermine, dans au moins un état de fonctionnement, la composante de la denrée alimentaire au moins au moyen d'un résultat d'une détection des éléments de capteur (22a, 24a).

5. Système de préparation de denrées alimentaires selon l'une des revendications précédentes, **caractérisé en ce que** l'unité de commande (18a) est conçue pour guider un utilisateur à travers au moins une recette et pour au moins tenir compte de la composante de la denrée alimentaire lors du guidage à travers la recette.

6. Système de préparation de denrées alimentaires selon l'une des revendications précédentes, **caractérisé en ce que** l'unité de commande (18a) délivre au moins une information dans au moins un état de fonctionnement en fonction de la détermination de la composante de la denrée alimentaire.

7. Système de préparation de denrées alimentaires selon l'une des revendications précédentes, **caractérisé en ce que** l'unité de commande (18a) délivre dans au moins un état de fonctionnement au moins une recommandation d'assaisonnement pour la denrée alimentaire.

8. Système de préparation de denrées alimentaires selon l'une des revendications précédentes, **caractérisé en ce que** l'unité de commande (18a) émet, dans au moins un état de fonctionnement, au moins une instruction de commande pour effectuer une dégustation de la denrée alimentaire.

9. Système de préparation de denrées alimentaires selon l'une des revendications précédentes, **caractérisé en ce que** l'unité de commande (18a) émet, dans au moins un état de fonctionnement, au moins une instruction de commande concernant au moins un retour d'information.

10. Système de préparation de denrées alimentaires selon l'une des revendications précédentes, **caractérisé en ce que** l'unité de commande (18a) est configurée pour détecter, dans au moins un état de fonctionnement, la composante de la denrée alimentaire à des intervalles de temps de 600 s au maximum au moyen de l'unité de détection (16a).

11. Système de préparation de denrées alimentaires selon l'une des revendications précédentes, **caractérisé par** au moins un appareil de cuisson (30a), qui comprend l'unité de commande (18a).
